# EUROPEAN PATENT APPLICATION

(11) **EP 4 371 488 A1**
(43) Date of publication of application: **22.05.2024**
(21) Application number: 22858756.4
(22) Date of filing: 17.08.2022
(51) Int. Cl.: A61B 5/327, A61B 5/346, A61B 5/28, A61B 5/00, G16H 50/20

(54) **SYSTEM FOR GENERATING PLURALITY OF PIECES OF STANDARD ELECTROCARDIOGRAM DATA USING 2-LEAD ELECTROCARDIOGRAM DATA**

(30) Priority: 17.08.2021 KR 20210107775
(71) Applicant: Medicalai Co., Ltd., Seoul 06302 (KR)
(72) Inventor: KWON, Joon Myoung, Seoul 06629 (KR)
(74) Representative: Isarpatent
(86) International application number: PCT/KR2022/012296
(87) International publication number: WO 2023/022519

(57) **Abstract**

Disclosed is a system for generating a plurality of pieces of standard electrocardiogram data using 2-lead electrocardiogram data, the system including: an electrocardiogram measurement unit (110) configured to generate electrocardiogram data by measuring 2- or more-lead standard lead electrocardiograms; an electrocardiogram generation unit (120) configured to generate multi-lead electrocardiogram data by identifying and computing the characteristic information of the 2- or more-lead standard lead electrocardiogram data and synthesizing a plurality of pieces of residual lead electrocardiogram data in a three-dimensional space through a pre-trained rule-based algorithm (121); and an output unit (130) configured to output the actual electrocardiogram data measured by the electrocardiogram measurement unit (110) and the multi-lead electrocardiogram data generated by the electrocardiogram generation unit (120), thereby generating a plurality of pieces of standard lead electrocardiogram data from 2- or more-lead electrocardiogram data using the rule-based algorithm (121).

## Description

### Technical Field

The present invention relates to a system for generating a plurality of pieces of standard electrocardiogram data using 2-lead electrocardiogram data, which may generate a plurality of pieces of standard lead electrocardiogram data from 2- or more-lead electrocardiogram data using a rule-based algorithm.

### Background Art

As is well known, since the development of an electrocardiogram, knowledge related to the electrocardiogram has expanded exponentially. Through electrocardiogram tests, information about the electrical functions of the heart can be obtained and various heart diseases such as arrhythmia, coronary artery disease, and myocardial disease can be diagnosed.

Recently, research into AI algorithms for electrocardiograms has been actively conducted. By AI algorithms, heart failure is detected, atrial fibrillation among arrhythmia rhythms is predicted, and gender is determined.

As described above, by overcoming human limitations, subtle changes in electrocardiogram waveforms can be detected and electrocardiogram interpretation can be further improved by AI algorithms.

Meanwhile, the electrocardiograms used in the medical field are 12-lead electrocardiograms. Such a 12-lead electrocardiogram is measured by attaching 10 electrodes, including 3 limb electrodes, 6 chest electrodes and 1 ground electrode, and the measured electrocardiogram data can be transmitted remotely.

However, it is inconvenient to expose the chest, attach 10 electrodes and then use a device in everyday life, so that a portable pad measurement device, a Galaxy Watch, an Apple Watch, or the like capable of measuring a single-lead electrocardiogram is also used.

A single-lead electrocardiogram measured as described above can be used to diagnose arrhythmia. However, a single-lead electrocardiogram has limitations in terms of use in the diagnosis of a disease requiring electrocardiogram information for various leads, such as myocardial infarction.

Therefore, there is a demand for technology that can easily measure 2- or more-lead asynchronous electrocardiograms using a single-lead electrocardiogram device or a 6-lead electrocardiogram device and predict a disease from the measured 2- or more-lead asynchronous electrocardiogram data.

### Disclosure

### Technical Problem

The technical problem to be overcome by the spirit of the present invention is to provide a system for generating a plurality of pieces of standard electrocardiogram data using 2-lead electrocardiogram data, which may generate a plurality of pieces of standard electrocardiogram data from 2- or more-lead electrocardiogram data using a rule-based algorithm.

### Technical Solution

In order to overcome the above technical problem, an embodiment of the present invention provides a system for generating a plurality of pieces of standard electrocardiogram data using 2-lead electrocardiogram data, the system including: an electrocardiogram measurement unit configured to generate electrocardiogram data by measuring 2- or more-lead standard lead electrocardiograms; an electrocardiogram generation unit configured to generate multi-lead electrocardiogram data by identifying and computing the characteristic information of the 2- or more-lead standard lead electrocardiogram data and synthesizing a plurality of pieces of residual lead electrocardiogram data in a three-dimensional space through a pre-trained rule-based algorithm; and an output unit configured to output the actual electrocardiogram data measured by the electrocardiogram measurement unit and the multi-lead electrocardiogram data generated by the electrocardiogram generation unit.

In this case, the rule-based algorithm may determine two coordinate axes corresponding to potential vectors by analyzing 2-lead electrocardiogram data accumulated in medical institutions and the direction information of the individual potential vectors of the 2-lead electrocardiogram data, may generate an additional potential vector by computing the two potential vectors to generate the 3D space, and may generate the multi-lead electrocardiogram data corresponding to the potential vector directions of respective pieces of standard lead electrocardiogram data by projecting the electrocardiogram data in the potential vector direction of the residual lead electrocardiogram data in the 3D space.

Furthermore, the rule-based algorithm may generate the additional electric potential vector by subtracting the two electric potential vectors from each other, and may generate the 3D space composed of the three electric potential vectors.

Furthermore, the rule-based algorithm may generate the 3D space by separating 3D components from the two potential vectors.

Furthermore, the rule-based algorithm may generate four-dimensional electrocardiogram data by adding a time axis to the 3D space using time-series information included in the electrocardiogram data.

Furthermore, the rule-based algorithm may visualize the coordinate axes of the 3D space constructed by the potential vectors and the additional potential vector or output the numerical information of the additional potential vector through a mathematical expression algorithm configured to extract the additional potential vector from the potential vectors, may calculate a coordinate axis corresponding to the potential vector of the residual lead electrocardiogram data, and may generate electrocardiogram data corresponding to the coordinate axis.

Moreover, the multi-lead electrocardiogram data may be synchronous lead electrocardiogram data, asynchronous lead electrocardiogram data, or lead electrocardiogram data generated without consideration of synchronization.

### Advantageous Effects

According to the present invention: a plurality of standard lead electrocardiograms may be generated in a 3D space using only the 2-lead electrocardiograms measured from an examinee, and thus a plurality of standard lead electrocardiograms used to diagnose and predict disease may be generated using only a small amount of electrocardiogram information and provided to medical staff; and a plurality of standard lead electrocardiograms may be generated using only a small amount of electrocardiogram information, and thus a health condition may be measured, diagnosed, examined, and predicted more accurately.

### Description of Drawings

FIG. 1 shows a block diagram of a system for generating a plurality of pieces of standard electrocardiogram data using 2-lead electrocardiogram data according to an embodiment of the present invention;
FIG. 2 is a diagram showing the generation of electrocardiogram data performed by the system for generating a plurality of pieces of standard electrocardiogram data using 2-lead electrocardiogram data shown in FIG. 1; and
FIG. 3 illustrates a flowchart of the generation of electrocardiograms performed by the system for generating a plurality of pieces of standard electrocardiogram data using 2-lead electrocardiogram data shown in FIG. 1.

### Mode for Invention

Embodiments of the present invention having the above-described features will be described in more detail below with reference to the accompanying drawings.

A system for generating a plurality of pieces of standard electrocardiogram data using 2-lead electrocardiogram data according to an embodiment of the present invention includes: an electrocardiogram measurement unit 110 configured to generate electrocardiogram data by measuring 2-or more-lead standard lead electrocardiograms; an electrocardiogram generation unit 120 configured to generate multi-lead electrocardiogram data by identifying and computing the characteristic information of the 2- or more-lead standard lead electrocardiogram data and synthesizing a plurality of pieces of residual lead electrocardiogram data in a 3D space through a pre-trained rule-based algorithm 121; and an output unit 130 configured to output the actual electrocardiogram data measured by the electrocardiogram measurement unit 110 and the multi-lead electrocardiogram data generated by the electrocardiogram generation unit 120. The gist of the system for generating a plurality of pieces of standard electrocardiogram data using 2-lead electrocardiogram data is to generate a plurality of pieces of standard lead electrocardiogram data from 2- or more-lead electrocardiogram data using the rule-based algorithm 121.

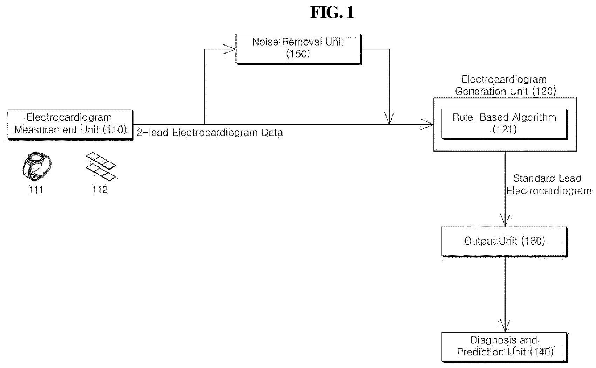

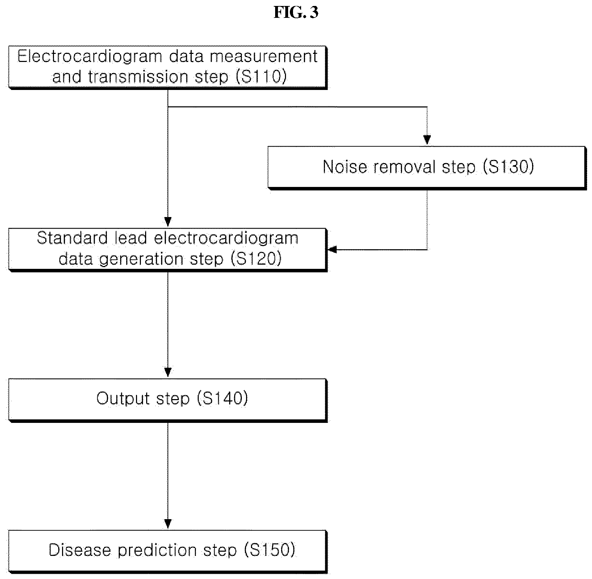

The system for generating a plurality of pieces of standard electrocardiogram data using 2-lead electrocardiogram data configured as described above will be described in detail below with reference to FIGS. 1 and 3 as follows.

First, the electrocardiogram measurement unit 110 is a component that measures 2- or more-lead standard electrocardiograms and then generates electrocardiogram data. The electrocardiogram measurement unit 110 is provided with two electrodes, acquires 2- or more-lead electrocardiogram data corresponding to at least two electrical axes in such a manner as to bring the two electrodes into contact with two portions of the body of an examinee, and then transmits the acquired 2- or more-lead electrocardiogram data to the electrocardiogram generation unit 120 through wired/wireless short-distance communication.

For example, a plurality of electrocardiograms for at least two different electrical axes may be measured by measuring a lead-I electrocardiogram corresponding to one electrical axis in such a manner as to bring the respective electrodes into contact with both hands and also measuring a lead-II electrocardiogram corresponding to the other electrical axis in such a manner as to bring the respective electrodes into contact with the right and left ankles, which are different from the previous body combination for contact with the electrodes.

Furthermore, the electrocardiogram measurement unit 110 may include a wearable electrocardiogram patch 111, a smartwatch 112 or a 6-lead electrocardiogram bar for short-term measurement capable of contact or contactless electrocardiogram measurement during daily life, or an electrocardiogram measurement device installed in a medical institution and configured to measure 2- or more-lead electrocardiograms, and may thus measure synchronous or asynchronous electrocardiograms.

In this case, the electrocardiogram measurement unit 110 may measure successive electrocardiograms of an examinee and transmit them to the electrocardiogram generation unit 120, or may measure two electrocardiograms at different times and transmit them to the electrocardiogram generation unit 120.

Next, through the pre-trained rule-based algorithm 121, the electrocardiogram generation unit 120 generates multi-lead electrocardiogram data by synthesizing a plurality of pieces of residual lead electrocardiogram data in a 3D space by identifying and computing the characteristic information of the 2- or more-lead standard lead electrocardiogram data.

For example, the rule-based algorithm 121 determines two coordinate axes corresponding to potential vectors by analyzing 2-lead electrocardiogram data, which is big data accumulated in the servers of medical institutions, and the direction information of the individual potential vectors, which are the characteristic information of the 2-lead electrocardiogram data, and generates an additional potential vector by computing the two potential vectors geometrically or mathematically, thereby generating a 3D space identical to the space where electrocardiograms are actually measured from the coordinate axes of the three potential vectors.

As described above, multi-lead electrocardiogram data corresponding to the potential vector directions of respective pieces of standard lead electrocardiogram data may be generated by projecting the electrocardiogram data in the direction of the potential vector of the residual lead electrocardiogram data in the 3D space where the 2-lead electrocardiogram data is present.

In other words, the rule-based algorithm 121 generates electrocardiograms that can be recorded in a plurality of various potential vector directions based on the electrocardiogram information determined in a 3D space. The rule-based algorithm 121 may be trained on training datasets of arbitrary 2-lead electrocardiogram data of big data, a 3D space generated by the computation of the 2-lead electrocardiogram data, and the residual multi-lead electrocardiogram data of the same examinee in the 3D space. Accordingly, the rule-based algorithm 121 may identify the type of lead electrocardiogram data by identifying the characteristic information of the electrocardiogram data from the 2-lead electrocardiogram data input from the electrocardiogram measurement unit 110, may generate a 3D space, and may project the potential record of the 2-lead electrocardiogram in the 3D space in various potential vector directions of a plurality of pieces of electrocardiogram data for leads not previously identified or a plurality of pieces of electrocardiogram data to be generated according to selection, thereby generating electrocardiogram data that can be recorded in individual lead electrocardiogram potential vector directions.

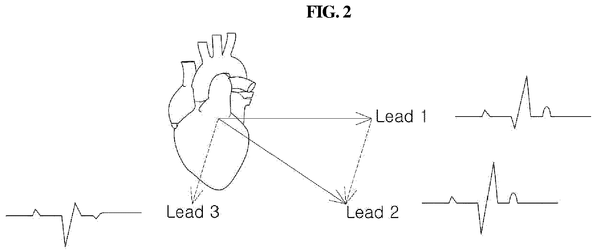

Meanwhile, referring to FIG. 2, the rule-based algorithm 121 generates an additional potential vector corresponding to electrocardiogram data of lead 3 by geometrically subtracting two potential vectors corresponding to electrocardiogram data of lead 1 and lead 2 from each other, thereby generating a 3D space composed of three potential vectors.

Alternatively, the rule-based algorithm 121 may generate a 3D space by separating 3D components from the two potential vectors, respectively. The rule-based algorithm 121 may determine the type of lead electrocardiogram data by identifying the characteristic information of the corresponding electrocardiogram data, may extract the 3D component of a corresponding potential vector, and may generate a 3D space from the extracted 3D information.

Furthermore, the rule-based algorithm 121 may generate four-dimensional (4D) electrocardiogram data by adding a time axis to the previously generated 4D space using the time-series information included in the electrocardiogram data, and may analyze and predict the trend of occurrence of disease and the trend of worsening or alleviation of the disease by analyzing electrocardiograms in a time-series manner through the time-series changes of the 3D electrocardiograms.

Furthermore, the rule-based algorithm 121 may geometrically compute, visualize and display the coordinate axes of the 3D space constructed by the two potential vectors provided from the electrocardiogram measurement unit 110 and the additionally generated potential vector, or may mathematically output the numerical information of the additional potential vector through a specific mathematical expression algorithm configured to extract an additional potential vector from potential vectors. Accordingly, a coordinate axis corresponding to the potential vector of the residual lead electrocardiogram data may be computed, and a potential record may be projected onto the coordinate axis so that recordable electrocardiogram data corresponding to the coordinate axis can be generated.

Furthermore, the electrocardiogram data measured by the electrocardiogram measurement unit 110 or the plurality of pieces of lead electrocardiogram data generated by the electrocardiogram generation unit 120 may be synchronous lead electrocardiogram data, asynchronous lead electrocardiogram data, or the lead electrocardiogram data generated without the consideration of synchronization.

Meanwhile, there may be further included a noise removal unit 150 in order to further increase the reliability of the standard lead electrocardiogram data generated by the electrocardiogram generation unit 120 by minimizing noise in the electrocardiogram data provided from the electrocardiogram measurement unit 110 to the electrocardiogram generation unit 120. For example, through the deep learning algorithm 121 constructed by being trained on training datasets of electrocardiogram data for individual leads having less noise and the unique styles of the electrocardiogram data for individual leads based on a large amount of electrocardiogram data, such as the standard 12-lead electrocardiogram data accumulated in medical institutions, the noise removal unit 150 may extract a corresponding unique style from the electrocardiogram data measured by the electrocardiogram measurement unit 110 with the characteristics of an examinee and the characteristics of a measurement method reflected therein, and may perform conversion into and generate electrocardiogram data of a specific lead style not containing noise through the extracted unique style.

Furthermore, the noise removal unit 150 may identify the unique style of each electrocardiogram data for each lead with high accuracy with the characteristics of the examinee attributable to age, gender, disease, etc. and the characteristics of the measurement method attributable to the attachment position of electrodes, an electrocardiogram device, etc. reflected therein, and may perform conversion into and generate electrocardiogram data for each lead based on the identified unique style more accurately.

Next, the output unit 130 may output and provide the actual electrocardiogram data measured by the electrocardiogram measurement unit 110 and the plurality of pieces of lead electrocardiogram data generated by the electrocardiogram generation unit 120 to medical staff.

Thereafter, the electrocardiogram data output from the output unit 130 is input to the diagnosis and prediction unit 140 constructed from standard lead electrocardiogram data and configured to predict disease, so that a health condition corresponding to the electrocardiogram data measured by the electrocardiogram measurement unit 110 can be diagnosed and predicted.

Meanwhile, the diagnosis and prediction unit 140 may diagnose and predict diseases of the circulatory system, endocrine, nutritional and metabolic diseases, neoplastic diseases, mental and behavioral disorders, diseases of the nervous system, diseases of the eyes and their appendages, diseases of the ears and mastoids, diseases of the respiratory system, diseases of the digestive system, diseases of the skin and skin tissue, diseases of the musculoskeletal system and compressive tissue, diseases of the genitourinary system, pregnancy, childbirth and postpartum diseases, and congenital anomalies, deformities and chromosomal abnormalities.

In addition, through the diagnosis and prediction unit 140, the damage caused by physical trauma may be identified, the prognosis thereof may be checked and the pain thereof may be measured, the risk of death or worsening attributable to trauma may be predicted, concurrent complications may be detected or predicted, and specific conditions that appear before or after birth may also be identified.

In addition, through the diagnosis and prediction unit 140, for the healthcare field, the health condition of an examinee, which can lead to services such as aging, sleep, weight, blood pressure, blood sugar, oxygen saturation, metabolism, stress, tension, fear, drinking, smoking, problem behavior, lung capacity, exercise volume, pain management, obesity, body mass, body composition, diet, type of exercise, lifestyle pattern recommendations, emergency management, chronic disease management, medication prescription, test recommendation, checkup recommendation, nursing, telehealth management, telemedicine, vaccination, and post-vaccination management, may be measured, diagnosed, examined, and predicted.

Not only health conditions attributable to the individual diseases described above but also complex health conditions may be measured, diagnosed, examined and predicted, the worsening or alleviation of the health condition of an examinee may be predicted, short-term and long-term prognoses may be predicted, and the state of metastasis or complication from one disease to another may be predicted. The improvement or deterioration of health conditions attributable to specific drugs and the analysis and prediction of electrocardiograms may be trained. A specific drug may be recommended according to a health condition.

FIG. 3 illustrates a flowchart of the generation of electrocardiograms performed by the system for generating a plurality of pieces of standard electrocardiogram data using 2-lead electrocardiogram data shown in FIG. 1, which will be briefly described in detail as follows.

First, through the electrocardiogram measurement unit 110, 2- or more-lead electrocardiogram data corresponding to at least two electrical axes is acquired in such a manner as to bring the two electrodes into contact with two portions of the body of an examinee and is then transmitted to the electrocardiogram generation unit 120 through wired/wireless short-distance communication in step S110.

Thereafter, by the electrocardiogram generation unit 120 provided with the pre-trained rule-based algorithm 121, multi-lead electrocardiogram data is generated by synthesizing a plurality of pieces of residual lead electrocardiogram data in a 3D space by identifying and computing the characteristic information of the 2- or more-lead standard lead electrocardiogram data in step S120.

For example, the rule-based algorithm 121 determines two coordinate axes corresponding to potential vectors by analyzing 2-lead electrocardiogram data, which is big data accumulated in the servers of medical institutions, and the direction information of the individual potential vectors, which are the characteristic information of the 2-lead electrocardiogram data, and generates an additional potential vector by computing the two potential vectors geometrically or mathematically, thereby generating a 3D space identical to the space where electrocardiograms are actually measured from the coordinate axes of the three potential vectors.

Meanwhile, there may be further included step S130 of removing, by the noise removal unit 150, noise from the electrocardiogram data in order to further increase the reliability of the standard lead electrocardiogram data generated by the electrocardiogram generation unit 120 by minimizing noise in the electrocardiogram data provided from the electrocardiogram measurement unit 110 to the electrocardiogram generation unit 120. For example, through the deep learning algorithm 121 constructed by being trained on training datasets of electrocardiogram data for individual leads having less noise and the unique styles of the electrocardiogram data for individual leads based on a large amount of electrocardiogram data, such as the standard 12-lead electrocardiogram data accumulated in medical institutions, the noise removal unit 150 may extract a corresponding unique style from the electrocardiogram data measured by the electrocardiogram measurement unit 110 with the characteristics of an examinee and the characteristics of a measurement method reflected therein, and may perform conversion into and generate electrocardiogram data of a specific lead style not containing noise through the extracted unique style.

Thereafter, through the output unit 130, the actual electrocardiogram data measured by the electrocardiogram measurement unit 110 and the plurality of pieces of lead electrocardiogram data generated by the electrocardiogram generation unit 120 may be output and provided to medical staff in step 140.

Thereafter, there may be further included step S150 of being constructed from standard lead electrocardiogram data and predicting disease by using the electrocardiogram data output from the output unit 130. Through the diagnosis and prediction unit 140, a health condition corresponding to the electrocardiogram data measured by the electrocardiogram measurement unit 110 may be diagnosed and predicted in step S150.

Therefore, according to the above-described configuration of the system for generating a plurality of pieces of standard electrocardiogram data using 2-lead electrocardiogram data: a plurality of standard lead electrocardiograms may be generated in a 3D space using only the 2-lead electrocardiograms measured from an examinee, and thus a plurality of standard lead electrocardiograms used to diagnose and predict disease may be generated using only a small amount of electrocardiogram information and provided to medical staff; and a plurality of standard lead electrocardiograms may be generated using only a small amount of electrocardiogram information, and thus a health condition may be measured, diagnosed, examined, and predicted more accurately.

The embodiments described in the present specification and the configurations shown in the drawings are only the most preferred embodiments of the present invention and do not represent the overall technical spirit of the present invention. Accordingly, it should be understood that at the time when the present application is filed, there may be various equivalents and modifications that can replace the above embodiments and/or the configurations.

## Claims

1. A system for generating a plurality of pieces of standard electrocardiogram data using 2-lead electrocardiogram data, the system comprising:
an electrocardiogram measurement unit configured to generate electrocardiogram data by measuring 2- or more-lead standard lead electrocardiograms;
an electrocardiogram generation unit configured to generate multi-lead electrocardiogram data by identifying and computing characteristic information of the 2- or more-lead standard lead electrocardiogram data and synthesizing a plurality of pieces of residual lead electrocardiogram data in a three-dimensional space through a pre-trained rule-based algorithm; and
an output unit configured to output the actual electrocardiogram data measured by the electrocardiogram measurement unit and the multi-lead electrocardiogram data generated by the electrocardiogram generation unit.

2. The system of claim 1, wherein the rule-based algorithm determines two coordinate axes corresponding to potential vectors by analyzing 2-lead electrocardiogram data accumulated in medical institutions and direction information of the individual potential vectors of the 2-lead electrocardiogram data, generates an additional potential vector by computing the two potential vectors to generate the 3D space, and generates the multi-lead electrocardiogram data corresponding to potential vector directions of respective pieces of standard lead electrocardiogram data by projecting the electrocardiogram data in a potential vector direction of the residual lead electrocardiogram data in the 3D space.

3. The system of claim 2, wherein the rule-based algorithm generates the additional electric potential vector by subtracting the two electric potential vectors from each other, and generates the 3D space composed of the three electric potential vectors.

4. The system of claim 2, wherein the rule-based algorithm generates the 3D space by separating 3D components from the two potential vectors.

5. The system of claim 3 or 4, wherein the rule-based algorithm generates four-dimensional electrocardiogram data by adding a time axis to the 3D space using time-series information included in the electrocardiogram data.

6. The system of claim 2, wherein the rule-based algorithm visualizes coordinate axes of the 3D space constructed by the potential vectors and the additional potential vector or outputs numerical information of the additional potential vector through a mathematical expression algorithm configured to extract the additional potential vector from the potential vectors, calculates a coordinate axis corresponding to a potential vector of the residual lead electrocardiogram data, and generates electrocardiogram data corresponding to the coordinate axis.

7. The system of claim 1, wherein the multi-lead electrocardiogram data is synchronous lead electrocardiogram data, asynchronous lead electrocardiogram data, or lead electrocardiogram data generated without consideration of synchronization.
